# EUROPEAN PATENT APPLICATION

(11) **EP 3 378 378 A1**
(43) Date of publication of application: **26.09.2018**
(21) Application number: 17161737.6
(22) Date of filing: 20.03.2017
(51) Int. Cl.: A61B 5/00, A61B 5/107

(54) **DEVICE, SYSTEM AND METHOD FOR MEASUREMENT OF A PROFILE OF A BODY PART**

(71) Applicant: Koninklijke Philips N.V., 5656 AE Eindhoven (NL)
(72) Inventor: JIN, Sheng (Kim), 5656 AE Eindhoven (NL); TIAN, Cong, 5656 AE Eindhoven (NL)
(74) Representative: de Haan, Poul Erik

(57) **Abstract**

The present invention relates to a device (1) for measurement of a profile of a body part, the device (1) comprising an elongated ruler (2) with markers (4), and a sensor (5) for measurement of data relating to a position and/or an orientation of the sensor (5) with respect to the ruler (2),, wherein the sensor (5) is slidably arranged on the ruler (2) and wherein the sensor (5) is in contact or engagement with the ruler (2).

## Description

### FIELD OF THE INVENTION

The present invention relates to a device for measurement of a profile of a body part. More specifically, the body part in question may be the abdomen of a pregnant woman. The invention further relates to a system comprising a device for measurement of the profile of the body part and a respective measurement method.

### BACKGROUND OF THE INVENTION

During pregnancy, expectant mothers would like to be informed about the condition of their babies. Information like fetal load, fetal size and fetal growth allow an assessment of the baby's growth and whether the development of the pregnancy proceeds according to predetermined values. However, nowadays the consultations at the obstetrician are rather short and leave pregnant women often in doubt whether they got all relevant information. Besides, pregnant women like to be informed about their weight gain so as to get back to shape after delivery.

In clinics, fetal growth and fetal size may be roughly screened by using the method of fundal height. Fundal height is a measurement method of the abdominal profile of a pregnant woman. It is measured from the top of the women's uterus to the top of the pubic symphysis. However, the method is not a very sensitive measurement for assessing the fetal size, since only one direction of the abdominal profile is assessed. The method has further disadvantages, which restrict the level of information detected by the method. On the one hand, fundal height does not provide information on the whole abdominal shape, which would provide more accurate information to estimate the fetal load, size and growth. On the other hand, fundal height is used predominantly by healthcare professionals or midwives. It is neither reliably nor conveniently to be used by the pregnant women themselves.

The state of the art presents approaches to measure certain parameters of a human body which may be adapted to collect data with respect to pregnancy. For example, document US 2014/0121564 A1 describes systems and methods for estimating body fat in a user with applications in the healthcare and personal health fields. An electronic device, such as a portable electronic device like a smartphone, a pad or a tablet may include software like an app to implement body fat estimates of a user and may use hardware and/or software resident in the electronic device like a display, an accelerometer, gyroscopes, transducers, vibration engines, speakers, microphones or GPS capability to aid a user in placing the electronic device at instructed locations on the user's body and to apply an impulse to the body at instructed locations.

However, most of such devices are not suitable to measure the abdominal profile of a pregnant woman, but are restricted to certain measurements mostly related to weight loss, which are not highest priority for a pregnant woman.

### SUMMARY OF THE INVENTION

It is an object of the present invention to provide a device for measurement of a profile of a body part, preferably of an abdomen of a pregnant woman, which allows an easy measurement with reasonably substantiated results with regard to the most significant parameters during pregnancy.

In a first aspect of the present invention a device for measurement of a profile of a body part is presented, the device comprising an elongated ruler with markers, and a sensor for measurement of data relating to a position and/or an orientation of the sensor. The sensor is slidably arranged on the ruler and in contact or engagement with the ruler.

In a further aspect of the present invention a system for measurement of a profile of a body part is presented, the system comprising a device according to one of the preceding claims, and a processing unit for determining a profile of a body part from the measured data.

In yet a further aspect of the invention, a method for measurement of a profile of a body part is presented, the method comprising the steps of arranging an elongated ruler with markers in a predetermined position on the body part, displacing a sensor along the length of the ruler over the markers, collecting data of the sensor during displacement of the sensor according to a position and/or an orientation of the sensor with respect to the ruler, calculating a partial profile of the body part from the collected data, and repeating the preceding steps in at least one different position and/or orientation of the ruler.

By way of this, a very easy and yet precise determination of the shape of the abdominal region of a pregnant woman may be conducted and conclusions about the pregnancy situation and the health and welfare of the unborn maybe deduced therefrom. The measurement may be carried out quickly and reliably even in remote areas with low medical supply since the components are easy to handle, small and lightweight, do not need external power supply or extended infrastructure. Pregnant women may consult the medical staff without preparations and will get reliable information about the course of pregnancy and the condition of the fetus immediately or at least on a very short time scale.

Alternatively, the measurement may also be carried out easily and conveniently at home when necessary, e.g. when women have to stay recumbent due to pregnancy complications and cannot attend medical consultations.

The inventive device and system may also be used for other body parts and in line with other applications especially in the area of fitness and health applications. The device may e.g. be used during a dietary period with the goal of losing weight or to shape body parts by selective exercises. The device then may be used to measure for example the dimension and shape of the belly and hips or limbs like thighs and so on and to monitor differences over time. As for pregnancy control, the use of the device and system is easy and may be carried out in various locations like gyms, at home or in hospitals.

It shall be understood that the claim method has similar and/or identical embodiments as the claimed device and as defined in the dependent claims.

Advantageously the ruler is bendable to adapt to the form of the body part. The measurement may be carried out easier with a flexible, bendable structure than with a rigid ruler. Besides, use of a flexible device will be accepted easier by a pregnant woman since the abdomen is often stressed and sensitive to pressure which should be avoided.

Preferably the markers on the ruler are spaced apart by predetermined distances, in particular the markers are spaced equidistantly. Especially equidistant markers facilitate the use of the device even by non-medical persons. The distances may also be chosen not to be equidistant but following a scale or pattern which may be translated to a rate or dimension.

The markers preferably protrude above a surface of the ruler which makes them easy to find for attachment of the sensor even if e.g. the light is dimmed. Likewise, a protruding shape helps to count the distance the sensor travels along the ruler.

According to an embodiment, the markers comprise sensors, in particular pressure sensors. These provide a cheap possibility to generate a signal directly connected to travelled distance when the sensor is moved along the ruler.

Advantageously, the sensor is arranged in a sliding component being mounted in contact or engagement with the ruler. If the sensor engages with the ruler, it cannot loose contact with the ruler and thus will deliver very reliable data when traveling along the length of the abdomen. Besides, the device may be easily stored when not used since the sensor cannot get lost.

According to an embodiment, the sensor is a three axes accelerometer sensor configured to measure changes of an angle between the direction of gravity and a direction defined by the movement of the sensor, or an inclination sensor configured to measure changes of an angle of inclination between a direction defined by an axis of the inclination sensor and a predetermined direction. Sensors incorporating accelerometers or inclination measurement devices nowadays are cheap and small and thus may be mounted in small sliding components which do not exert forces or pressure to the respective body part under measurement.

The processing unit which collects the data and evaluates the profile therefrom is arranged in or on the sliding component or alternatively in a mobile device.

Preferably the mobile device is a mobile phone, a tablet or a laptop. These devices are widespread and maybe equipped with apps or other software to analyze the collected data and display the results to the user.

In carrying out the method, it is preferred to place the ruler at different positions on the body part. After a first measurement at least one different position and/or orientation of the ruler is adapted which is parallel or rectangular to the first position. In this way, a number of values may be collected which offer the possibility to determine the shape of the body part in detail.

In particular, in an embodiment the processing unit is configured to determine a profile of a body part from measured changes of an angle between the direction of gravity and a direction that is defined by the movement of the sensor at different positions of the sensor. The distance between markers is generally known and provides information about the position at which the sensor measures data, e.g. because the sensor can recognize when it moves over a marker. The markers may alternatively be used as an indication at which position a measurement shall be taken, e.g. at which position the sensor may be stopped to take a measurement.

A total profile of the body part is preferably calculated from the partial profiles of the body part. By use of interpolation a three-dimensional virtual model may be generated. The shape of the model will inform the obstetrician or other medical staff about the situation at a higher degree of accuracy and thus helps to streamline treatment and information.

### BRIEF DESCRIPTION OF THE DRAWINGS

These and other aspects of the invention will be apparent from and elucidated with reference to the embodiment(s) described hereinafter. In the following drawings
Fig. 1 shows a view of application of a measurement method according to the state of the art,
Fig. 2 shows an embodiment of a device and a system according to the invention, and
Fig. 3A-3C show exemplary measurements according to the invention.

### DETAILED DESCRIPTION OF THE INVENTION

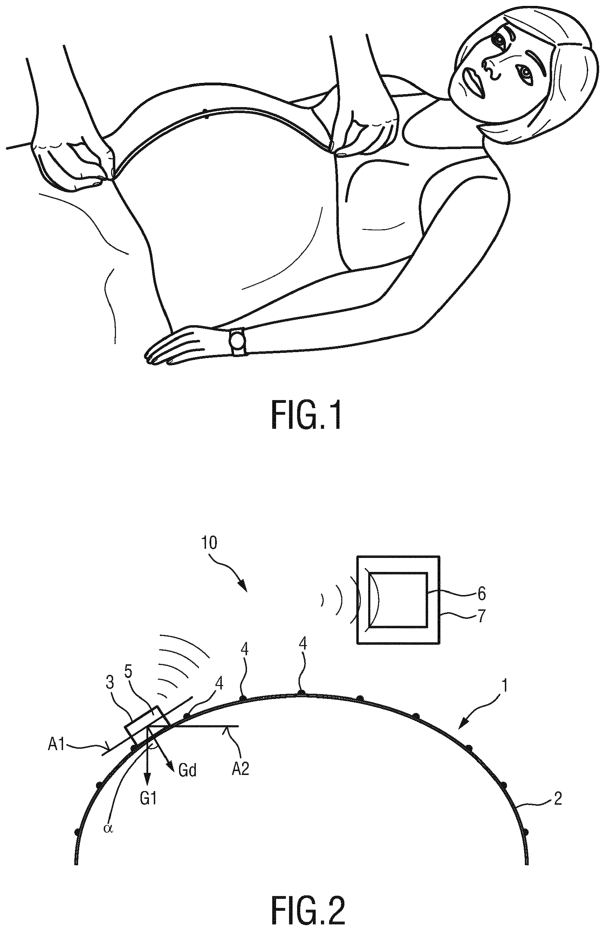

Fig. 1 shows a picture of above referenced measurement method of fundal height. This state of the art measurement method is widespread, especially in regions where other diagnostic equipment like ultrasonic equipment is scarce, not available or expensive. As may be seen in Fig. 1, the fundal height is measured from the top of the woman's uterus to the top of the pubic symphysis. The fundal height, when expressed in centimeters, roughly corresponds to gestational age in weeks between week 16 and week 36 for a normally orientated fetus. When a tape measure as shown in Fig. 1 is unavailable, finger widths are used to estimate centimeter respectively week distances from a corresponding anatomical landmark. However, landmark distances from the public symphysis are highly variable depending on body type and shape of the pregnant woman. In clinical practice, recording the actual fundal height measurement is a standard practice beginning at around 20 weeks of gestation. Most caregivers will record their patients' fundal height on every prenatal visit.

However, the state of the art measurement method is prone to inaccurateness due to several reasons. The method e.g. does not work any more shortly before delivery since the fetus descends into the pelvis. Besides, other factors like errors in estimated date of pregnancy, orientation of the fetus, physical differences between different women and their unborns and different diseases might influence the measurement. Besides, the measurement does not yield correct results for multiple pregnancies like twins, triplets or more.

As may be seen in Fig. 1, the measurement tape is placed on the abdomen of the pregnant woman and one measurement is carried out. This measurement only yields one length value, which is compared to reference values. It is directly apparent that this is neither sufficiently reliable nor satisfactory for the pregnant woman.

Thus, there is a demand for a better measurement method which is easy to use and may also be used by the pregnant woman herself.

In Fig. 2, a preferred embodiment of a device 1 and a system 10 according to the invention is shown in a schematically way. The device 1 according to the invention comprises an elongated ruler 2 which is flexible or bendable to adapt to the form of the body part, especially to the form of the abdomen of the pregnant woman. The bendable ruler 2 preferably consists of a flexible material like plastics or a thin metal strip. Since the ruler 2 is bendable, an additional track holder which is not shown in Fig. 2 maybe used to facilitate the measurement and to stabilize the ruler 2 in its actual position.

The ruler 2 contains markers 4 which are arranged in or on the ruler 2 and which are spaced apart by predetermined distances, in particular by equidistant distances. The markers 4 maybe attached to the ruler 2, especially when the materials of the ruler 2 and the markers 4 are different. Alternatively, the ruler 2 and the markers 4 may be in one piece. This may be easily achieved when the ruler 2 and the markers are of the same material. In Fig. 2, the markers 4 protrude over a surface of the ruler 2, but it would likewise be possible to form the markers 4 as recesses in the ruler 2.

The choice of the markers 4 depends on the shape and functionality of a sliding component 3, which is slidably arranged on the ruler 2. The sliding component 3 is at least in contact or even in engagement with the ruler 2. Engagement is preferred since it helps to keep the sliding component 3 on the ruler 2, makes measurement easier and prevents loss of the sliding component 3 when the device 1 is not in use and stored away.

The sliding component 3 contains at least one sensor 5 which may be for example an accelerometer sensor configured to measure changes of an angle α between the direction of gravity G1 and a direction Gd that is defined by the movement of the sensor 5. In particular, in the embodiment shown in Fig. 2, the direction Gd is perpendicular to the longitudinal direction A1, which the movement direction of the sensor 5 (or to the tangent direction of the ruler 2 at the position of the sensor). Hereby, the angle α maybe determined by arccos(gd/G1), where gd is the acceleration value in the direction Gd.

Alternatively, an inclination sensor maybe used which is configured to measure changes of an angle of inclination between a direction defined by a longitudinal axis A1 of the inclination sensor and a predetermined direction A2. The latter possibility does not imply that the pregnant woman has to lie down. Combinations of more than one sensor 5 and/or different types of sensors 5 are possible.

The markers 4 may also comprise sensors, in particular pressure sensors, to facilitate placement of the sliding component 3 on the ruler 2 and to more reliable detect the movement of the sliding component 3.

The system 10 may further comprise a processing unit 6, which is adapted to receive and process signals sent from the at least one sensor 5 in the sliding component 3 and/or from the sensors of the markers 4. The processing unit 6 maybe arranged in the sliding component 3 or alternatively, as shown in Fig. 2, in an external device as part of the system 10, which may e.g. be a mobile device 7.

The proposed method will be described in the following. As mentioned above, the measurement takes place via use of the at least one sensor 5, the bendable ruler 2 and the processing unit 6. The processing unit 6 is configured to reconstruct the shape and size of the abdomen of the pregnant woman. First, the ruler 2 is put on the abdomen along its curve at a certain predetermined position. Positioning may be guided e.g. by the mobile device 7 or by a chart showing the positions. The mobile device 7 may be for example a mobile phone having a mobile app. After positioning the ruler 2 along the abdomen of the pregnant woman, a sliding component 3 containing the at least one sensor 5 may be put on certain positions on the ruler 2. Preferably, the operation is checked and confirmed by evaluating starting data, e.g. the angle of the accelerometer to the gravity direction and the sensor's position on the ruler 2. These values may be recorded by the sensor 5 itself or wirelessly transferred to the processing unit 6 in the mobile device 7, which may process the measured data to compute a partial profile (e.g. the profile along one direction) of the abdomen.

With several repetitions of this operation on different positions of the ruler 2, which may for example include parallel and/or rectangular arrangements of the ruler 2 with respect to the initial position and orientation of the ruler 2, a series of data comprising positon and angle information will be recorded and analyzed to calculate a shape and size profile (i.e. a total profile) of the abdomen along the ruler's 2 direction. With the ruler on different positions on the abdomen and by interpolation, an approximated three-dimensional abdominal profile may be reconstructed. Since the distance between any two markers 4 are known and the two angles against gravity of any two markers 4 are known, the profile of any curve derived from each measurement position of the ruler 2 is known. If multiple curves are known, for instance, a vertical one and a horizontal one from top view, then the whole profile of the abdomen can be rebuilt by interpolation between these curves with the prior knowledge that the whole profile should have a half-sphere like shape. As an embodiment, 5 positions and according orientations may constitute a very accurate curve with prior knowledge of a general model of the body part determined on a population level.

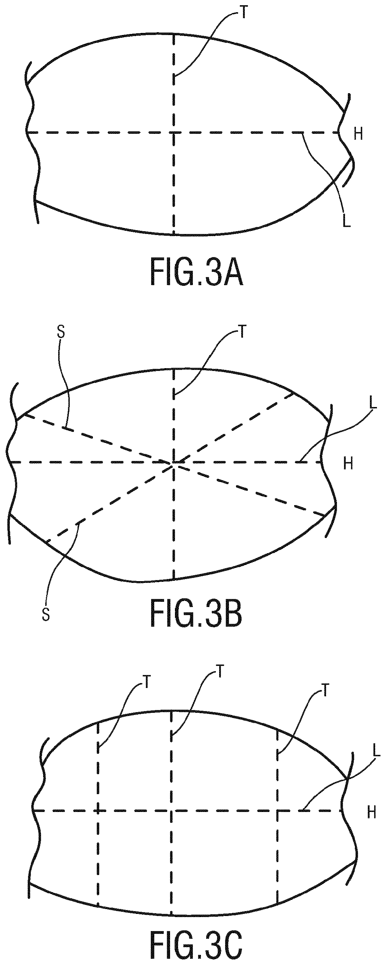

As described exemplarily by Figs. 3A to 3C, the positions of the ruler 2 preferably follow a grid with perpendicular orientation of the different measurement positions. In Figs. 3A to 3C, a very schematic top view of the belly of the pregnant woman is shown. H denotes the position of the head of the person. The dotted lines mark the different positions of the ruler 2 during measurement. Fig. 3A shows a simple measurement with one measurement along a transversal body axis T and one along a longitudinal body axis L. In Fig. 3B additionally two measurements along skew lines S are carried out. The skew lines S contribute to the three-dimensional body model calculated from the measurements.

Particular positioning may be selected according to different interests, where more positions of the ruler 2 will be allocated in the body part with higher resolution requirement. In Fig. 3C for example a higher resolution will be achieved in horizontal direction, i.e. there is one measurement carried out along the longitudinal body axis L and three measurements are carried out along the transversal body axis T in three different positions of the ruler 2. Reference locations may also be set as a standard to follow to secure a more accurate positioning, where reference locations shall be covered by the curves determined by the sensors 5.

Alternatively to the accelerometer sensor, an activity watch with integrated three axis accelerometer may be used. A button or other components which may be touched or operated by the fingers indicates that the sliding component is ready being placed in the correct position. Alternatively, an automatic routine may detect the presence of the ruler 2. The acceleration information and position which are indicated by a counter which counts the number of times the button is pushed maybe recorded in an internal storage of the system 10 or may be wirelessly transferred to the mobile device 7. The button may be operated manually or alternatively by way of the aforementioned pressure sensors in the markers 4. When the watch slides along the ruler 2, the counts are then recorded. This may be used as additional information to the three-dimensional abdominal profile, so that a higher resolution of the particular of interest may be achieved.

The mobile device 7 may be a smartphone with a mobile app which may guide the user through the steps of putting the ruler 2 in the correct position and orientation. The mobile device 7 records the information of position and acceleration data and calculates the abdominal profile therefrom. The mobile device 7 will guide the user how to position the ruler 2 correctly. For instance, an end of the ruler 2 should be positioned at the center of the abdomen's left or right side. For the use of the accelerometer sensor, the user should lay down to measure the profile due to the angle being calculated by a gravity projection. From two measurements of the ruler on different positions, which may be also crossing positions, the profile may be calculated by interpolation and fitting. The profile then may be used to derive other parameters like fetal size, gestational weight gain etc.

The sliding component 3 slides from one end of the ruler 2 to the other end across the whole abdomen, while the angle is continuously measured and the distance the sensor 5 in the sliding component 3 walks is recorded by way of the markers 4 set on the ruler 2. The distance between the markers 4 is fixed as a distance unit, and the markers 4 may be realized as a round protrusion, which cause rapid changes of signal of the accelerometer thus indicating the markers 4. Based on the counting of the markers 4, the distance the sensor 5 walks with the sliding component 3 maybe determined. Markers 4 may also be implemented as additional sensors like pressure sensors to provide similar information to the processing unit 6.

With the distance the sensor 5 walks and the angle change the accelerometer experiences, an abdominal shape curve maybe determined. After multiple curves are determined, e.g. horizontal and vertical curves, a rough 3D model of the abdomen may be established. The more curves are determined the more accurate the resulting profile will be.

In a special embodiment, the sensor 5 and the sliding component 3 may be implemented in a watch, where the ruler 2 may be a specially designed belt and the accelerometer sensor is in the watch itself. The respective watch may be used for other measurements for example in the health sector.

While the invention has been illustrated and described in detail in the drawings and foregoing description, such illustration and description are to be considered illustrative or exemplary and not restrictive; the invention is not limited to the disclosed embodiments. Other variations to the disclosed embodiments may be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Device (1) for measurement of a profile of a body part, the device (1) comprising:
- an elongated ruler (2) with markers (4), and
- a sensor (5) for measurement of data relating to a position and/or an orientation of the sensor (5) with respect to the ruler(2),
wherein the sensor (5) is slidably arranged on the ruler (2) and wherein the sensor (5) is in contact or engagement with the ruler (2).

2. Device (1) according to claim 1, wherein the ruler (2) is bendable to adapt to the form of the body part.

3. Device (1) according to claim 1, wherein the markers (4) are spaced apart by predetermined distances, in particular the markers (4) are spaced equidistantly.

4. Device according to claim 3, wherein the markers (4) protrude above a surface of the ruler (2).

5. Device according to claim 3 or 4, wherein the markers (4) comprise sensors, in particular pressure sensors.

6. Device (1) according to claim 1, wherein the sensor (5) is arranged in a sliding component (3) being mounted in contact or engagement with the ruler (2).

7. Device (1) according to claim 1, wherein the sensor (5) is an accelerometer sensor, in particular a three axes accelerometer sensor, configured to measure changes of an angle between the direction of gravity (G1) and a direction defined by the movement (Gd) of the sensor (5) or an inclination sensor configured to measure changes of an angle of inclination between a direction defined by an axis (A1) of the inclination sensor and a predetermined direction (A2).

8. System (10) for measurement of a profile of a body part, the system (10) comprising:
- a device (1) according to one of the preceding claims, and
- a processing unit (6) for determining a profile of a body part from the measured data.

9. System (10) according to claim 8, wherein the processing unit (6) is arranged in or on the sliding component (3) or in a mobile device (7).

10. System (10) according to claim 8 or 9, wherein the mobile device (7) is a mobile phone, a tablet or a laptop.

11. System (10) according to claim 8 or 9, wherein the processing unit (6) is configured to determine a profile of a body part from measured changes of an angle (α) between the direction of gravity (G1) and a direction (Gd) that is defined by the movement of the sensor (5) at different positions of the sensor (5).

12. Method for measurement of a profile of a body part, the method comprising the steps of:
- arranging an elongated ruler with markers in a predetermined position on the body part,
- moving a sensor along the length of the ruler over the markers,
- collecting data of the sensor during displacement of the sensor according to a position and/or an orientation of the sensor with respect to the ruler,
- calculating a partial profile of the body part from the collected data, and
- repeating the preceding steps in at least one different position and/or orientation of the ruler.

13. Method according to claim 12, wherein the at least one different position and/or orientation of the ruler is parallel or rectangular to the first position.

14. Method according to claim 12 or 13, wherein a total profile is calculated from the partial profiles by use of interpolation.

15. Device (1), system (10) and method according to one of the preceding claims, wherein the body part is an abdomen of a pregnant woman.
